# EUROPÄISCHE PATENTANMELDUNG

(11) **EP 1 671 615 A2**
(43) Veröffentlichungstag der Anmeldung: **21.06.2006**
(21) Anmeldenummer: 05026680.8
(22) Anmeldetag: 07.12.2005
(51) Int. Cl.: A61K 8/06, A61K 8/43, A61Q 5/00, A61Q 17/00, A61Q 19/00

(54) **Verwendung von Alkylguanidinen als kationische Emulgatoren**

(30) Priorität: 20.12.2004 US 17589
(71) Anmelder: Goldschmidt GmbH, 45127 Essen (DE)
(72) Erfinder: Fötsch, Hannelore, 45355 Essen (DE); Howe, Anna, Moseley Virginia 23120 (US); Meyer, Jürgen, Dr., 48143 Münster (DE); Pascaly, Matthias, Dr., 48163 Münster (DE)

(57) **Zusammenfassung**

Ein Gegenstand der Erfindung sind Emulsionen, enthaltend kationische Emulgatoren und gegebenenfalls Co-Emulgatoren, gegebenenfalls übliche Hilfs- und Zusatzstoffe, welche dadurch gekennzeichnet sind, dass als kationische Emulgatoren mindestens eine Verbindung der allgemeinen Formel (I) 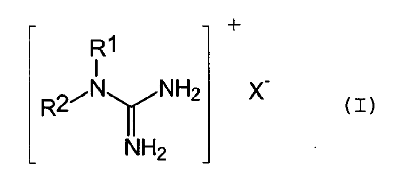 worin
- R¹,R²: unabhängig voneinander mindestens ein Rest ist ausgesucht aus der Gruppe
H, und/oder ein gegebenenfalls verzweigter, gegebenenfalls Mehrfachbindungen enthaltender Kohlenwasserstoffrest, ein Hydroxyalkyl-, Alkoxyalkyl-, Alkylpolyalkoxyalkyl-, Carboxyalkyl-Rest, ein Aminoalkyl-, Alkylaminoalkyl-, Amidoalkyl-, Alkylamidoalkyl-Rest, ein homo- oder hetrocyclischer, gegebenenfalls substituierter aliphatischer oder aromatischer Kohlenwasserstoffrest mit 1 bis 60 C-Atomen, vorzugsweise 1 bis 30 C-Atomen, insbesondere 12 bis 18 C-Atomen, mit der Maßgabe, dass die Summe der C-Atome aus R¹ + R² mindestens 12 ist und
- X: ein salzbildendes Anion oder das Hydroxylanion ist,
verwendet wird.

## Beschreibung

Die vorliegende Erfindung betrifft die Verwendung von Alkylguanidinen als kationische Emulgatoren, insbesondere zur Herstellung von Emulsionen für kosmetische, dermatologische oder pharmazeutische Zubereitungen.

Emulsionen stellen einen wichtigen Produkttyp im Bereich kosmetischer, dermatologischer und/oder pharmazeutischer Zubereitungen dar.

Kosmetische Zubereitungen werden im Wesentlichen zur Hautpflege benutzt. Hautpflege im kosmetischen Sinn ist in erster Linie, dass die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen und die Weichheit und Glätte der Haut zu erhalten bzw. wieder herzustellen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern. Die Summe dieser Hautpflegeeffekte wird in der Kosmetik allgemein unter dem Begriff "skin conditioning" zusammengefasst.

Kosmetische Zubereitungen werden auch als Desodorantien verwendet. Pharmazeutische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

Durch den steten Anstieg des Anteils älterer Menschen in der Bevölkerung tritt zunehmend bei kosmetischen Formulierungen die feuchtigkeitsspendende Wirkung (moisturizing) dieser Präparate in den Vordergrund, da insbesondere ältere Menschen unter trockener Haut leiden. Dieser Moisturizing-Effekt wird durch kosmetisch akzeptable Filme auf der Haut erzielt, die den transepidermalen Wasserverlust der Haut an die Atmosphäre unterbinden und stattdessen Wasser in der stratum corneum Schicht anreichern.

Eine der Möglichkeiten, diese Problemstellung mit Hilfe von kosmetischen Formulierungen anzugehen, ist die Verwendung von Pflegeemulsionen mit einem hohen Ölphasengehalt. Dies kann bei der Verwendung üblicher nichtionischer oder ionischer Emulgatoren allerdings dazu führen, dass derartige Formulierungen ein sehr öliges, fettiges und schweres Hautgefühl mit sich bringen.

Kationische Emulsionen auf Basis hautverträglicher kationischer Emulgatoren bewirken ein extrem trockenes Hautgefühl, was dazu benutzt werden kann die oben beschriebenen Effekte hoher lipophiler Beladung zu maskieren. Dieser Sachverhalt hat in den letzten Jahren dazu geführt, dass ein verstärktes Interesse an kationischen Emulsionen auf dem Kosmetikmarkt zu beobachten ist (A. Paez, A. Howe, Cosmetics and Toiletries Manufacture Worldwide, May 2004, 67-71).

Kombinationen aus einer Ölphase enthaltend beispielsweise ein oder mehrere Komponenten wie Mineralöl, Vaseline, Weißöl, Cetylalkohol, Isopropylpalmitat in Kombination mit kationischen Emulgatoren wie beispielsweise Fettalkyldimethylbenzylammoniumverbindungen sind bekannt aus den Patentschriften US 3 666 690; US 3 818 105; US 4 137 302.

Aus der EP-B-0 058 853 sind Hautpflegezusammensetzungen zum Befeuchten und Konditionieren der Haut (Moisterizing und Conditioning) mit verbesserten taktilen Eigenschaften (Hautgefühl) bekannt, enthaltend eine Ölphase und quartäre Ammoniumverbindungen der allgemeinen Formel 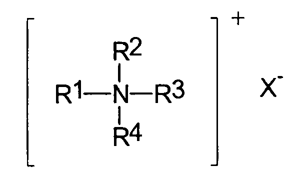 worin
- R¹,R²: im Wesentlichen lineare Alkylketten mit 16 bis 22 C-Atomen,
- R³,R⁴: Alkylreste mit 1 bis 3 C-Atomen und
- X⁻: ein salzbildendes Anion bedeuten.

Als Emulgator werden bevorzugt quartäre Ammoniumverbindungen wie Distearyldimoniumchlorid genannt.

Nachteilig bei diesen Verbindungen ist jedoch, dass sie zur Erzielung stabiler Emulsionen in relativ großen Mengen, typischerweise 3 bis 6 Gew.-% bezogen auf die Formulierung, dieser quartären Ammoniumverbindungen eingesetzt werden müssen und sie in diesen Konzentrationen bei häufiger Anwendung allergische oder auf Überempfindlichkeit des Anwenders beruhende Reaktionen hervorrufen können, sowie ihre nach heutigen Gesichtspunkten unzureichende biologische Abbaubarkeit.

Weiterhin wird oft das zwar trockene, aber in vielen Fällen doch sehr stumpfe Hautgefühl beklagt, das Emulsionen auf Basis dieser quartären Ammoniumverbindungen auszeichnet.

Es bestand daher ein Bedarf an Emulgatoren zur Herstellung von Emulsionen insbesondere für kosmetische, dermatologische oder pharmazeutische Zubereitungen, die bereits in deutlich geringeren Konzentrationen stabile Emulsionen bilden, keine Irritationen auf der Haut hervorrufen und ökologisch unbedenklich sind und die - auch bei alleiniger Verwendung - kein negativ empfundenes, stumpfes Hautgefühl hervorrufen.

Überraschenderweise wurde nun gefunden, dass langkettige Alkylguanidine (Alkylkettenlängen von C₁₂-C₃₀) und deren Salze diese Anforderungen erfüllen.

Zwar ist die Verwendung von kurzkettigen Alkylguanidinen (C₁₋C₁₀) in Hautpflegeprodukten in der Literatur beschrieben (US-A 5 723 133, US-A-5 939 078), doch wird dort lediglich auf die physiologischen Wirkungen dieser Substanzen (als sogenannte active ingredients) eingegangen. Die Verwendung von Alkylguanidinen als leistungsstarke Emulgatoren wird an keiner Stelle beschrieben und war somit für den Fachmann nicht vorhersehbar.

Als erfindungsgemäße Emulgatoren kommen vor allem Alkylguanidine und deren Salze der Kettenlänge C₁₂-C₆₀, vorzugsweise C₁₂₋C₃₀ in Frage, von denen sich insbesondere Oleyl-, Stearyl-, Oleyl-/Stearyl-, Dioleyl-, Distearylguanidiniumsalze durch ihre hervorragenden emulgieraktiven Eigenschaften bei der Herstellung von kosmetischen O/W-Emulsionen auszeichnen.

Die erfindungsgemäß verwendeten oder mitverwendeten Alkylguanidiniumsalze besitzen sowohl eine gute Stabilität als auch eine gute Formulierbarkeit, rufen bereits in geringen Einsatzkonzentrationen eine deutliche Wirkung hervor, sind nicht toxisch, naturnah, biologisch gut abbaubar, werden sehr gut von der Haut toleriert, weisen eine hohe Verträglichkeit mit anderen Inhaltsstoffen auf und lassen sich problemlos einarbeiten. Zusätzlich weisen sie eine leicht antimikrobielle Wirkung auf.

Ihre herausragende Emulgieraktivität beweisen sie dadurch, dass sie bereits mit gegenüber dem Stand der Technik deutlich geringeren Mengen, nämlich 0,05 bis 3 Gew.-% vorzugsweise 0,1 bis 3 Gew.-% und insbesondere 0,2 bis 1,5 Gew.-% Alkylguanidin, bezogen auf die Formulierung, ohne Zusatz weiterer emulgieraktiver Stoffe stabile O/W-Formulierungen bilden. Höhere Konzentrationen bis zu 10 Gew.-% sind für spezielle Verwendungszwecke und zur zusätzlichen Erzielung weiterer Wirkungen aber durchaus möglich.

Weiterhin können die erfindungsgemäßen Alkylguanidine auch in nahezu jeder beliebigen Konzentration als Co-Emulgatoren für O/W- und W/O-Formulierungen eingesetzt werden.

Das Hautgefühl, das durch den Einsatz dieser Alkylguanidine erzielt werden kann, ähnelt den klassischen kationischen Emulgatoren, ist allerdings in vielen Fällen weniger stumpf und somit deutlich angenehmer.

Ein Gegenstand der Erfindung sind daher Emulsionen, enthaltend kationische Emulgatoren und gegebenenfalls Co-Emulgatoren, gegebenenfalls übliche Hilfs- und Zusatzstoffe, welche dadurch gekennzeichnet sind, dass als kationische Emulgatoren mindestens eine Verbindung der allgemeinen Formel (I) 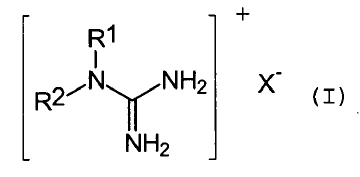 worin
- R¹,R²: unabhängig voneinander mindestens ein Rest ist ausgesucht aus der Gruppe H, und/oder ein gegebenenfalls verzweigter, gegebenenfalls Mehrfachbindungen enthaltender Kohlenwasserstoffrest, ein Hydroxyalkyl-, Alkoxyalkyl-, Alkylpolyalkoxyalkyl-, Carboxyalkyl-Rest, ein Aminoalkyl-, Alkylaminoalkyl-, Amidoalkyl-, Alkylamidoalkyl-Rest, ein homo- oder hetrocyclischer, gegebenenfalls substituierter aliphatischer oder aromatischer Kohlenwasserstoffrest mit 1 bis 60 C-Atomen, vorzugsweise 1 bis 30 C-Atomen, insbesondere 12 bis 18 C-Atomen, mit der Maßgabe, dass die Summe der C-Atome aus R¹ + R² mindestens 12 ist und
- X⁻: ein salzbildendes Anion oder das Hydroxylanion ist,
verwendet wird.

Ein weiterer Gegenstand der Erfindung ist die Verwendung der Emulsionen zur Herstellung von kosmetischen, dermatologischen oder pharmazeutischen Zubereitungen.

Ein weiterer Gegenstand der Erfindung ist die Verwendung der Emulsionen zur Herstellung von kosmetischen Reinigungs- und Pflegezubereitungen für Haut- und Hautanhangsgebilde.

Ein weiterer Gegenstand der Erfindung ist die Verwendung der Emulsionen zur Herstellung von Sonnenschutzformulierungen.

Ein weiterer Gegenstand der Erfindung ist die Verwendung der Emulsionen zur Herstellung von Desodorantien und Antitranspirantien.

Ein weiterer Gegenstand der Erfindung ist die Verwendung der Emulsionen zur Herstellung von Zubereitungen zur Behandlung und Nachbehandlung von Geweben auf Basis natürlicher oder synthetischer Fasern wie insbesondere zur Erstausrüstung von Textilien oder, gegebenenfalls in Kombination mit quartären Verbindungen, als Wäscheweichspülmittel.

Ein weiterer Gegenstand der Erfindung ist die Verwendung der Emulsionen zur Herstellung von Reinigungs- und Pflegemitteln für harte Oberflächen.

Ein weiterer Gegenstand der Erfindung ist die Verwendung von Alkylguanidinen und deren Salze in Öl-in-Wasser- oder in Wasser-in-Ö1-Emulsionen in einer Konzentration von üblicherweise 0,05 bis 10 Gew.-%, vorzugsweise 0,1 bis 5 Gew.-%, insbesondere 0,2 bis 3 Gew.-%.

Ein weiterer Gegenstand der Erfindung ist die Verwendung von Alkylguanidinen und deren Salze als alleinige Emulgatoren in kosmetischen und pharmazeutischen Öl-in-Wasser-Emulsionen.

Ein weiterer Gegenstand der Erfindung ist die Verwendung von Alklyguanidinen und deren Salze als Co-Emulgatoren in kosmetischen und pharmazeutischen Öl-in-Wasser-Emulsionen.

Ein weiterer Gegenstand der Erfindung ist die Verwendung von Alkylguanidinen und deren Salze als Co-Emulgatoren in kosmetischen und pharmazeutischen Wasser-in-Öl-Emulsionen.

Die zur Herstellung der erfindungsgemäß verwendeten Alkylguanidine eingesetzten Fettamine können nach bekannten Verfahren z.B. durch Umsetzung von Fettsäuren mit NH₃ in Gegenwart von Katalysatoren zum Nitril und anschließender Hydrierung zum primären Amin hergestellt werden.

Derartige Amine werden aus einzelnen oder Mischungen der Fettsäuren wie Caprylsäure, Caprinsäure, 2-Ethylhexansäure, Laurinsäure, Myristinsäure, Palmitinsäure, Palmitoleinsäure, Isostearinsäure, Stearinsäure, Hydroxystearinsäure (Ricinolsäure), Dihydroxystearinsäure, Ölsäure, Linolsäure, Petroselinsäure, Elaidinsäure, Arachinsäure, Behensäure und Erucasäure, Gadoleinsäure sowie die bei der Druckspaltung natürlicher Fette und Öle anfallenden technischen Mischungen wie Ölsäure, Linolsäure, Linolensäure und insbesondere Rapsölfettsäure, Sojaölfettsäure, Sonnenblumenölfettsäure, Tallölfettsäure, Kokosöl erhalten. Geeignet sind prinzipiell alle Fettsäuren mit ähnlicher Kettenverteilung. Der Gehalt dieser Fettsäuren bzw. Fettsäureester an ungesättigten Anteilen, wird - soweit dies erforderlich ist - durch die bekannten katalytischen Hydrierverfahren auf eine gewünschte Jodzahl eingestellt oder durch Abmischung von vollhydrierten mit nichthydrierten Fettkomponenten erzielt.

Vorzugsweise werden teilgehärtete C₈₋₁₈-Kokos- bzw. Palmfettsäuren, Rapsölfettsäuren, Sonnenblumenölfettsäuren Sojaölfettsäuren und Tallölfettsäuren, mit Jodzahlen im Bereich von ca. 80 bis 150 und insbesondere technische C₈₋₁₈-Kokos-fettsäuren eingesetzt, wobei gegebenenfalls eine Auswahl von cis/trans-Isomeren wie elaidinsäurereiche C_{16/18}-Fettsäure-schnitte von Vorteil sein können. Sie sind handelsübliche Produkte und werden von verschiedenen Firmen unter deren jeweiligen Handelsnamen angeboten.

Die erfindungsgemäß verwendeten Alkylguanidiniumsalze weisen demnach eine mittlere Alkylkettenlänge von C₁₂-C₆₀, vorzugsweise C₁₂-C₃₀, insbesondere C₁₂-C₁₈ auf.

Alkylguanidiniumsalze der Formel I liegen im Gleichgewicht mit den nicht protonierten Alkylguanidinen vor, wobei die Lage des Gleichgewichts naturgemäß durch den pH-Wert der Zubereitung bestimmt wird.

Zur Salzbildung geeignet sind grundsätzlich alle kosmetisch unbedenklichen anorganischen oder organischen ein- oder mehrbasischen Säuren wie beispielsweise Ameisensäure, Propionsäure, Heptansäure, Caprylsäure, Nonansäure, Caprinsäure, Undecansäure, Laurinsäure, Myristinsäure, Palmitinsäure, Stearinsäure, Arachinsäure, Behensäure, Cyclopentancarbonsäure, Cyclohexancarbonsäure, Acrylsäure, Methacrylsäure, Vinylessigsäure, Crotonsäure, 2-/3-/4-Pentensäure, 2-/3-/4-/5-Hexensäure, Lauroleinsäure, Myristoleinsäure, Palmitoleinsäure, Ölsäure, Gadoleinsäure, Sorbinsäure, Linolsäure, Linolensäure, Pivalinsäure, Ethoxyessigsäure, Phenylessigsäure, 2-Ethylhexansäure, Oxalsäure, Glycolsäure, Malonsäure, Bernsteinsäure, Glutarsäure, Adipinsäure, Pimelinsäure, Korksäure, Azelainsäure, Sebacinsäure, Benzoesäure, o-/m-/p-Tolylsäure, Salicylsäure, 3-/4-Hydroxybenzoesäure, Phthalsäuren, oder deren ganz oder teilweise hydrierten Derivate wie Hexahydro- oder Tetrahydrophthalsäure und deren Gemische, insbesondere Kohlensäure, Phosphorsäure, Schwefelsäure, Essigsäure, Milchsäure, Weinsäure, Äpfelsäure, Citronensäure. Dabei ist es im Sinne der vorliegenden Erfindung auch möglich, sowohl geeignete Guanidin-Derivate untereinander in Mischungen zu verwenden als auch Mischsalze.

Eine Möglichkeit zur Herstellung der erfindungsgemäß verwendeten Alkylguanidine ist beispielsweise in der DE-506 282 beschrieben. In dem Verfahren werden Alkylamine in einer alkoholischen Lösung mit Cyanamid in Anwesenheit einer Protonensäure guanidyliert. So werden die Produkte als kristalline Salze erhalten.

Weiterer Bestandteil der Erfindung ist der Einsatz von Alkylguanidinen und deren Salze zusammen mit einem oder mehreren anderen Emulgatoren. Diese Verwendung der Alkylguanidine als Co-Emulgatoren ist dabei prinzipiell sowohl in Ö1-in-Wasser (O/W)- als auch in Wasser-in-Öl (W/O)-Emulsionen möglich.

Insbesondere betrifft dieser Einsatz als Co-Emulgator kosmetische Hautpflegeprodukte, in denen durch den Einsatz der erfindungsgemäßen Alkylguanidine oder deren Salze zum einen die Emulgieraktivität unterstützt wird und zum anderen ein spezielles Hautgefühl vermittelt wird, das dieser Substanzklasse zu eigen ist. Dieses Hautgefühl kann als sehr trocken beschrieben werden, zeichnet sich aber im Vergleich zu "klassischen" kationischen Emulgatoren dadurch aus, dass es deutlich weniger stumpf wirkt.

Werden zusätzlich zu Alkylguanidinen und deren Salzen weitere Emulgatoren verwendet, so kommen dafür beispielsweise nichtionogene Tenside aus mindestens einer der folgenden Gruppen in Frage:
- Anlagerungsprodukte von 2 bis 100 mol Ethylenoxid und/oder 0 bis 5 mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe
- C_{12/18}-Fettsäuremono- und -diester von Anlagerungsprodukten von 1 bis 100 mol Ethylenoxid an Glycerin
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte
- Alkylmono- und -oligoglycoside mit 8 bis 22 Kohlenstoffatomen im Alkylrest und deren ethoxylierte Analoga
- Anlagerungsprodukte von 2 bis 100 mol Ethylenoxid an Ricinusöl und/oder gehärtetes Ricinusöl
- Polyol- und insbesondere Polyglycerinester, wie z. B. Polyglycerinpolyricinoleat, Polyglycerin-12-hydroxystearat oder Polyglycerindimerat. Ebenfalls geeignet sind Gemische von Verbindungen aus mehreren dieser Substanzklassen
- Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose)
- Mono-, Di- und Trialkylphosphate sowie Mono-, Di- und/oder Tri-PEG-alkylphosphate und deren Salze
- Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 14/12 oder 20/20 oder 18/18 oder 17/18 oder 15/15. Besonders geeignet sind hierbei Produkte wie Bis-PEG/PPG-14/14 Dimethicone (mit Cyclopentasiloxan: ABIL® EM 97) oder insbesondere PEG/PPG-16/16 Dimethicone (mit Capryl/Caprin-Triglycerid: ABIL® Care 85)
- Polysiloxan-Polyalkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL® EM 90)
- Mischester aus Pentaerythrit, Fettsäuren, Citronensäure und Fettalkohol gemäß DE-1 165 574 und/oder Mischester von Fettsäuren mit 6 bis 22 Kohlenstoffatomen, Methylglucose und Polyolen, vorzugsweise Glycerin oder Polyglycerin sowie
- Polyalkylenglykol
- amphotere und zwitterionische Tenside wie Betaine
- anderen kationische Emulgatoren, wie z.B. Alkylquats, Esterquats, Siliconquats, sowie ethoxylierte Varianten davon.

Die Anlagerungsprodukte von Ethylenoxid und/oder von Propylenoxid an Fettalkohole, Fettsäuren, Alkylphenole, Glycerinmono- und diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Cocosalkyldimethylammoniumglycinat, N-Acylaminopropyl-N,N-dimethylammoniumglycinate, beispielsweise das Cocosacylaminopropyldimethylammoniumglycinat und 2-Alkyl-3-carboxyl-methyl-3-hydroxyehtylimidazoline mit jeweils 8 bis 18 C-Atomen in der Alkyl- oder Acylgruppe sowie das Cocosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind amphotere Tenside. Unter amphotere Tenside werden solche oberflächenaktiven Verbindungen verstanden, die außer einer C_{8/18}-Alkyl- oder -Acylgruppe im Molekül mindestens eine freie Aminogruppe und mindestens eine -COOH-oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte amphotere Tenside sind das N-Cocosalkylaminopropionat, das Cocosacylaminoethylaminopropionat und das C_{12/18}-Acylsarcosin. Neben den amphoteren kommen auch quartäre Emulgatoren in Betracht, wobei solche vom Typ der Esterquats, vorzugsweise methylquaternierte Difettsäuretriethanolaminester-Salze, besonders bevorzugt sind.

Weiterhin können die erfindungsgemäßen kationischen Emulsionen auf Basis von Alkylguanidinen und deren Salzen übliche Hilfs- und Zusatzstoffe enthalten wie Konsistenzgeber, Verdickungsmittel, Öle, Wachse, UV-Lichtschutzfilter, Antioxidantien, Hydrotrope, Deodorant- und Antitransparantwirkstoffe, Konservierungsmittel, Insektrepellentien, Selbstbräuner, Parfümöle, Farbstoffe und biogene Wirkstoffe.

Als Konsistenzgeber kommen in erster Linie Fettalkohole oder Hydroxyfettalkholole mit 12 bis 22 und vorzugsweise 16 bis 18 Kohlenstoffatomen und daneben Partialglyceride, Fettsäuren oder Hydroxyfettsäuren in Betracht.

Geeignete Verdickungsmittel sind beispielsweise Polysaccharide, insbesondere Xanthan-Gum, Guar, Agar-Agar, Alginate und Tylosen, Carboxymethylcellulose und Hydroxyethylcellulose, alkylmodifizierte Zuckerderivate wie z.B. Cetylhydroxyethylcellulose, ferner höhermolekulare Polyethylenglycolmono- und diester von Fettsäuren, Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als Ölphase kommen beispielsweise solche Ölkomponenten in Frage, die als kosmetische und pharmazeutische Ölkomponenten sowie als Gleit- und Schmiermittelkomponenten bekannt sind. Hierzu zählen insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen. Ebenso sind im erfindungsgemäßen Sinne die Veresterungsprodukte aliphatischer, difunktioneller Alkohole mit 2 bis 36 C-Atomen mit monofunktionellen aliphatischen Carbonsäuren mit 1 bis 22 C-Atomen geeignet. Als Ölkomponenten geeignete Monoester sind z. B. die Methylester und Isopropylester von Fettsäuren mit 12 bis 22 C-Atomen wie z. B. Methyllaurat, Methylstearat, Methyloleat, Methylerucat, Isopropylpalmitat, Isopropylmyristat, Isopropylstearat, Isopropyloleat in Frage. Andere geeignete Monoester sind z. B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z. B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z. b. im Jojobaöl oder im Spermöl vorliegen.

Geeignete Dicarbonsäureester sind z. B. Di-n-butyl-adipat, Di-n-butyl-sebacat, Di-(2-ethylhexyl)-adipat, Di-(2-hexyldecyl)-succinat, Di-isotridecylacetat. Geeignete Diolester sind z. B. Ethylenglycoldioleat, Ethylenglycol-di-isotridecanoat, Propylenglycol-di-(2-ethylhexanoat), Butandioldi-isostearat und Neopentylglycol-di-caprylat.

Weitere Fettsäureester, die eingesetzt werden können, sind z.B. C₁₂₋₁₅-Alkylbenzoate, Dicaprylylcarbonate, Diethylhexylcarbonate.

Ebenso als Ölkomponente geeignet sind die Fettsäuretriglyceride, wobei unter diesen die natürlich vorkommenden Öle und Fette bevorzugt sind. So kommen beispielsweise natürliche, pflanzliche Öle, z. B. Olivenöl, Sonnenblumenöl, Sojaöl, Erdnußöl, Rapsöl, Mandelöl, Palmöl aber auch die flüssigen Anteile des Kokosöls oder des Palmkernöls sowie tierische Öle wie z. B. Klauenöl, die flüssigen Anteile des Rindertalgs oder auch synthetische Triglyceride von Capryl-Caprinsäure-Gemischen, Triglyceride aus technischer Ölsäure oder aus Palmitinsäure-Ölsäure-Gemischen als Ölkomponenten in Frage.

Weiterhin können Kohlenwasserstoffe, insbesondere auch flüssige Paraffine und Isoparaffine eingesetzt werden. Weiterhin sind auch Fettalkohole wie Oleylalkohol oder Octyldodecanol, sowie Fettalkoholether wie Dicapryl-Ether einsetzbar.

Geeignete Siliconöle und -wachse sind z.B. Polydimethylsiloxane, Cyclomethylsiloxane, sowie aryl- oder alkyl- oder alkoxy-substituierte Polymethylsiloxane oder Cyclomethylsiloxane.

Insgesamt können die erfindungsgemäßen Formulierungen 1 bis 50 % Ölphase enthalten.

Unter UV-Lichtschutzfiltern sind organische Substanzen zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen sind z. B. zu nennen:
- 3-Benzylidencampher und dessen Derivate, z. B. 3-(4-Me-thylbenzyliden)camphor
- 4-Aminobenzoesäurederivate, vorzugsweise 4-(Dimethylamino)benzoesäure-2-ethylhexylester, 4-(Dimethylamino)benzoesäure-2-ethylhexylester und 4-(Dimethylamino)benzoe-säureamylester
- Ester der Zimtsäure, vorzugsweise 4-Methoxyzimtsäure-2-ethylhexylester, 4-Methoxyzimtsäureisopentylester, 2-Cyan-3-phenyl-zimtsäure-2-ethylhexylester (Octocrylene)
- Ester der Salicylsäure, vorzugsweise Salicylsäure-2-ethylhexylester, Salicylsäure-4-isopropylbenzylester, Salicylsäurehomomenthylester
- Derivate des Benzophenons, vorzugsweise 2-Hydroxy-4-methoxybenzophenon, 2-Hydroxy-4-methoxy-4'-methylbenzophe-non, 2,2'-Dihydroxy-4-methoxybenzophenon
- Ester der Benzalmalonsäure, vorzugsweise 4-Methoxy-benzalmalonsäuredi-2-ethylhexylester
- Triazinderivate, wie z. B. 2,4,6-Trianilino-(p-carbo-2'-ethyl-1'-hexyloxy)-1,3,5-triazin und Octyltriazon.
- Propan-1,3-dione, wie z. B. 1-(4-tert.Butylphenyl)-3-(4'methoxyphenyl)propan-1,3-dion.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium-und Glucammoniumsalze
- Sulfonsäurederivate von Benzophenon, vorzugsweise 2-Hydroxy-4-methoxybenzophenon-5-sulfonsäure und ihre Salze
- Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'tert.-Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von kleiner 200 nm, das z. B. als 50 %ige wässrige Dispersion erhältlich ist.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P. Finkel in SÖFW-Journal 122, 543 (1996) zu entnehmen.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton
- Technische Oligoglyceringemische mit einem Eigenkondensationsgrad von 1,5 bis 10 wie etwa technische Diglyceringemische mit einem Diglyceringehalt von 40 bis 50 Gew.-%
- Methylolverbindungen, wie insbesondere Trimethylolethan, Trimethylolpropan, Trimethylolbutan, Pentaerythrit und Dipentaerythrit
- Niedrigalkylgucoside, insbesondere solche mit 1 bis 8 Kohlenstoffatomen im Alkylrest, wie beispielsweise Methyl-und Butylglucosid
- Zuckeralkohole mit 5 bis 12 Kohlenstoffatomen, wie beispielsweise Sorbit oder Mannit
- Zucker mit 5 bis 12 Kohlenstoffatomen, wie beispielsweise Glucose oder Saccharose
- Aminozucker, wie beispielsweise Glucamin

Als Deodorantwirkstoffe kommen z. B. Geruchsüberdecker wie die gängigen Parfümbestandteile, Geruchsabsorber, beispielsweise die in der Patentoffenlegungsschrift DE-A-40 09 347 beschriebenen Schichtsilikate, von diesen insbesondere Montmorillonit, Kaolinit, Ilit, Beidelit, Nontronit, Saponit, Ilectorit, Bentonit, Smectit, ferner beispielsweise Zinksalze der Ricinolsäure in Frage. Keimhemmende Mittel sind ebenfalls geeignet, in die erfindungsgemäßen Öl-in-Wasser-Emulsionen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbonilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-A-198 55 934, DE-A-37 40 186, DE-A-39 38 140, DE-A-42 04 321, DE-A-42 29 707, DE-A-42 29 737, DE-A-42 38 081, DE-A-43 09 372, DE-A-43 24 219 beschriebenen wirksamen Agenzien. Weitere übliche Antitranspirantwirkstoffe können ebenfalls vorteilhaft in den erfindungsgemäßen Zubereitungen verwendet werden, insbesondere Adstringentien, beispielsweise basische Aluminiumchloride wie Aluminiumchlorhydrat ("ACH") und Aluminium-Zirkonium-Glycin-Salze ("ZAG").

Als Konservierungsmittel eignen sich beispielsweise Phenoxyethanol, Formaldehydlösung, Parabene, Pentandiol oder Sorbinsäure. Als Insekten-Repellentien kommen N,N-Diethyl-m-toluamid, 1,2-Pentandiol oder Insect Repellent 3535 in Frage, als Selbstbräuner eignen sich z.B. Dihydroxyaceton und Erythrulose, als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z. B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hautpsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 - 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Phytosphingosin (und Phytosphingosinderivate), Pseudoceramide, essentielle Öle, Pflanzenextrakte und Vitaminkomplexe zu verstehen.

### Herstellungsbeispiele:

### Darstellung von Laurylguanidiniumacetat:

44,1 g Laurylamin (0,238 mol) und 150 ml n-Butanol werden vorgelegt und bei 60 °C gelöst. In der Wärme wird dann 14,2 g Essigsäure (99,8 %; 0,237 mol) zugegeben. Danach stellt man eine Temperatur von 90 °C ein und tropft innerhalb von 3 Stunden das in 50 ml n-Butanol gelöste Cyanamid (11,0 g; 0,262 mol) zu. Nach Ende der Zugabe lässt man 3 Stunden bei 90 °C nachreagieren. Anschließend wird das Lösungsmittel am Rotationsverdampfer abgezogen. Zur Aufreinigung wird das Rohprodukt in Aceton aufgeschlämmt, filtriert, und der Rückstand mit Diethylether gewaschen. Restmengen Diethylether werden am Rotationsverdampfer bei vermindertem Druck entfernt. Es wird ein farbloses, kristallines Pulver erhalten.

Laurylguanidiniumacetat : ¹³C-NMR, 100 MHz, MeOD, 25°C: δ = 179,1 (1C, COOH_{AcOH}), 157,7 (1C, C_{Guanidiniumgr.}), 41,2 (1C, CH₂), 31, 6 (1C, CH₂), 29, 3 (4C, CH₂), 28,9 (2C, CH₂), 28, 6 (1C, CH₂), 26,4 (1C, CH₂), 23,2 (1C, CH₂), 22,3 (1C, CH_{3AcOH}), 13,1 (1C, CH₃); MALDI-TOF[m/z]: 228 (M+H⁺)

### Darstellung von Stearylguanidiniumacetat und Stearylguanidiniumlactat:

76,35 g Stearylamin (0,269 mol) und 150 ml n-Butanol werden vorgelegt und bei 60 °C gelöst. In der Wärme wird dann 16,19 g Essigsäure (0,269 mol) bzw. 26,95 g Milchsäure (0,269 mol) zugegeben. Danach stellt man eine Temperatur von 90 °C ein und tropft innerhalb von 3 Stunden das in 50 ml n-Butanol gelöste Cyanamid (11,31 g) zu. Nach Ende der Zugabe lässt man 3 Stunden bei 90 °C nachreagieren. Anschließend wird das Lösungsmittel am Rotationsverdampfer abgezogen. Zur Aufreinigung wird das Rohprodukt in Aceton aufgeschlämmt, filtriert und der Rückstand mit Diethylether gewaschen. Restmengen Diethylether werden am Rotationsverdampfer bei vermindertem Druck entfernt. Es wird ein weißes, kristallines Pulver erhalten.

Stearylguanidiniumacetat : ¹³C-NMR, 100 MHz, MeOD, 25°C: δ = 180,1 (1C, COOH_{AcOH}), 158, 8 (1C, C_{Guanidiniumgr.}), 42,2 (1C, CH₂), 32, 8 (1C, CH₂), 30,6 (12C, CH₂), 29, 7 (1C, CH₂), 27,6 (1C, CH₂), 24,6 (1C, CH₂), 23,5 (1C, CH_{3AcOH}), 14, 5 (1C, CH₃); MALDI-TOF[m/z] : 312 (M+H⁺)

Stearylguanidiniumlactat: ¹³C-NMR, 100 MHz, MeOD, Toluol-d, 50 ° C : δ = 181,7 (1C, COOH_{LacOH}), 157, 9 (1C, C_{Guanidiniumgr.}), 68,7 (1C, CH_{LacOH}), 41,6 (1C, CH₂), 32,1 (1C, CH₂), 29, 5 (12C, CH₂), 29, 1 (1C, CH₂), 26, 9 (1C, CH₂), 22,8 (1C, CH₂), 20,9 (1C, CH_{3LacOH}), 13,8 (1C, CH₃); MALDI-TOF[m/z]:

### Darstellung von Distearylguanidiniumlactat:

143,28 g Distearylamin (0,269 mol) und 150 ml n-Butanol werden vorgelegt und bei 60 °C gelöst. In der Wärme wird dann 26,95 g Milchsäure (0,269 mol) zugegeben. Danach stellt man eine Temperatur von 90 °C ein und tropft innerhalb von 3 Stunden das in 50 ml n-Butanol gelöste Cyanamid (11,31 g) zu. Nach Ende der Zugabe lässt man 3 Stunden bei 90 °C nachreagieren. Anschließend wird das Lösungsmittel am Rotationsverdampfer abgezogen. Zur Aufreinigung wird das Rohprodukt in Aceton aufgeschlämmt, filtriert und der Rückstand mit Diethylether gewaschen. Restmengen an Diethylether werden am Rotationsverdampfer bei vermindertem Druck entfernt. Es wird ein weißes, kristallines Pulver erhalten.

Distearylguanidiniumlactat : ¹³C-NMR, 100 MHz, MeOD, 50°C: δ = 182,2 (1C, COOH_{LacOH}), 157 , 5 (1C, C_{Guanidinogr.}), 69,2 (1C, CH_{LacOH}), 32,7 (2C, CH₂), 30,5 (28C, CH₂), 28,2 (2C, CH₂), 23,4 (2C, CH₂), 21,7 (1C, CH_{3,LacOH}), 14,5 (2C, CH₃); MALDI-TOF[m/z]:

### Anwendungsbeispiele:

Die folgenden Beispiele sollen den Gegenstand der Erfindung näher erläutern, ohne ihn darauf einzuschränken. Die Beispiele 1 bis 8 zeigen dabei die Verwendung von Alkylguanidinen als alleinige Emulgatoren, während die Beispiele 9 bis 18 den Einsatz von Alkylguanidinen als Co-Emulgatoren veranschaulichen. Die Konzentrationsangaben in allen Beispielen sind als Gew.-% angegeben.

### Emulsionen 1 bis 4:

Die Emulsionen 1 bis 4 sollen insbesondere zeigen, dass typische kationische O/W-Cremes bereits mit sehr geringen Emulgatorkonzentrationen von 0,5 bis 1,5 % Stearylguanidin oder auch Distearylguanidin erhältlich sind. Dabei war insbesondere überraschend, dass bereits bei Emulgatorkonzentrationen von 0,5 % Stearylguanidiniumacetat stabile Emulsionen erhältlich sind (6 Monate Stabilität bei Raumtemperatur, 45 °C und Gefriertauzyklen (3 x -15 °C)).

| | Emulsion | 1 % | 2 % | 3 % | 4 % |
|---|---|---|---|---|---|
| A | Glycerinstearat | 4,0 | 4,0 | 4,0 | 4,0 |
| | Stearylalkohol | 2,0 | 2,0 | 2,0 | 2,0 |
| | Capryl/Caprin-Triglycerid | 9,0 | 9,0 | 9,0 | 9,0 |
| | Mineralöl (30 mPas) | 8,0 | 8,0 | 8,0 | 8,0 |
| B | Glycerin | 3,0 | 3,0 | 3,0 | 3,0 |
| | Stearylguanidinium-Acetat | 0,5 | 1,0 | 1,5 | |
| | Distearylguanidinium-Acetat | | | | 1,5 |
| | Wasser | ad 100 | ad 100 | ad 100 | ad 100 |
| | Konservierungsmittel, Parfüm | q. s. | q. s. | q. s. | q. s. |

### Emulsionen 5 bis 8:

Die Emulsionen 5 bis 8 zeigen, dass stabile Emulsionen auch mit typischen anderen Emollientkombinationen möglich sind. Weiterhin veranschaulichen Emulsionen 7 bis 8 auch die hervorragenden emulgieraktiven Eigenschaften anderer Alklyguanidiniumsalze.

| | Emulsion | 5 % | 6 % | 7 % | 8 % |
|---|---|---|---|---|---|
| A | Glycerinstearat | 4,0 | 4,0 | 4,0 | 4,0 |
| | Stearylalkohol | 2,0 | 2,0 | 2,0 | 2,0 |
| | Capryl/Caprin-Triglycerid | 8,0 | | 8,0 | |
| | C₁₂₋₁₅ Alkyl-Benzoat | 9,0 | | 9,0 | |
| | Diethylhexyl-Carbonat | | 9,0 | | 9,0 |
| | Cyclopentasiloxan | | 8,0 | | 8,0 |
| B | Glycerin | 3,0 | 3,0 | 3,0 | 3,0 |
| | Stearylguanidinium-Acetat | 1,0 | 1,0 | | |
| | Stearylguanidinium-Lactat | | | 1,0 | 1,0 |
| | Wasser | ad 100 | ad 100 | ad 100 | ad 100 |
| | Konservierungsmittel, Parfüm | q. s. | q. s. | q. s. | q. s. |

### Emulsionen 9 bis 12:

Die Emulsionen 9 bis 12 zeigen die Verwendung erfindungsgemäßer Alkylguanidiniumsalze als Co-Emulgatoren in Kombination mit typischen nichtionischen Emulgatoren zur Einstellung eines sehr trockenen, aber nicht stumpfen Hautgefühls.

| | Emulsion | 9 % | 10 % | 11 % | 12 % |
|---|---|---|---|---|---|
| A | Ceteareth-25 | 1,5 | 1,5 | 1,5 | 1,5 |
| | Stearylalkohol | 2,0 | 2,0 | 2,0 | 2,0 |
| | Glycerinstearat | 4,0 | 4,0 | 4,0 | 4,0 |
| | Capryl/Caprin-Triglycerid | 8,0 | 8,0 | 8,0 | 8,0 |
| | Cetearyl-Ethylhexanoat | 8,5 | 8,5 | 8,5 | 8,5 |
| B | Glycerin | 3,0 | 3,0 | 3,0 | 3,0 |
| | Stearylguanidinium-Acetat | 1,0 | | | |
| | Stearylguanidinium-Lactat | | 1,0 | | |
| | Laurylguanidinium-Acetate | | | 1,0 | |
| | Laurylguanidinium-Lactat | | | | 1,0 |
| | Wasser | ad 100 | ad 100 | ad 100 | ad 100 |
| | Konservierungsmittel, Parfüm | q. s. | q. s. | q. s. | q. s. |

### Emulsionen 13 bis 15:

Die Emulsionen 13 bis 15 dienen insbesondere zur Veranschaulichung der Kombination von Alkylguanidinen mit Silicon-O/W-Emulgatoren. Diese Kombination zeichnet sich durch ein ausgezeichnetes trockenes und seidiges Hautgefühl aus.

| | Emulsion | 13 % | 14 % | 15 % |
|---|---|---|---|---|
| A | Bis-PEG/PPG-16/16 PEG/PPG 16/16 Dimethicone; Capryl/Caprin-Triglycerid (ABIL® Care 85) | 1,5 | 1,5 | 1,5 |
| | Stearylalkohol | 2,0 | 2,0 | 2,0 |
| | Glycerinstearat | 4,0 | 4,0 | 4,0 |
| | Capryl/Caprin-Triglycerid | 8,0 | 8,0 | 8,0 |
| | Cetearyl-Ethylhexanoat | 8,5 | 8,5 | 8,5 |
| B | Glycerin | 3,0 | 3,0 | 3,0 |
| | Stearylguanidinium-Acetat | 1,0 | | |
| | Laurylguanidinium-Acetat | | 1,0 | |
| | Distearylguanidinium-Acetat | | | 1,0 |
| | Wasser | ad 100 | ad 100 | ad 100 |
| | Konservierungsmittel, Parfüm | q. s. | q. s. | q. s. |

### Emulsionen 16 bis 18:

Die Emulsionen 16 bis 18 sollen insbesondere zeigen, dass der Einsatz von Alkylguanidinen als Co-Emulgatoren auch in W/O-Emulsionen möglich ist.

| | Emulsion | 16 % | 17 % | 18 % |
|---|---|---|---|---|
| A | Cetyl PEG/PPG-10/1 Dimethicone (ABIL® EM 90) | 2,0 | 2,0 | 2,0 |
| | Microkristallin-Wax | 1,2 | 1,2 | 1,2 |
| | hydriertes Castoröl | 0,8 | 0,8 | 0,8 |
| | Capryl/Caprin-Triglycerid | 5,0 | 5,0 | 5,0 |
| | Diethylhexyl-Carbonat | 7,0 | 7,0 | 7,0 |
| | Cetearyl-Ethylhexanoat | 7,0 | 7,0 | 7,0 |
| B | NaCl | 0,5 | 0,5 | 0,5 |
| | Stearylguanidinium-Acetat | 0,3 | | |
| | Stearylguanidinium-Lactat | | 0,3 | |
| | Laurylguanidinium-Acetat | | | 0,3 |
| | Wasser | ad 100 | ad 100 | ad 100 |
| | Konservierungsmittel, Parfüm | q. s. | q. s. | q. s. |

## Patentansprüche

1. Emulsionen, enthaltend kationische Emulgatoren und gegebenenfalls Co-Emulgatoren, gegebenenfalls übliche Hilfs-und Zusatzstoffe, **dadurch gekennzeichnet, dass** als kationische Emulgatoren mindestens eine Verbindung der allgemeinen Formel (I) 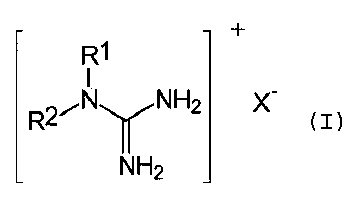 worin
R¹, R² unabhängig voneinander mindestens ein Rest ist ausgesucht aus der Gruppe H, und/oder ein gegebenenfalls verzweigter, gegebenenfalls Mehrfachbindungen enthaltender Kohlenwasserstoffrest, ein Hydroxyalkyl-, Alkoxyalkyl-, Alkylpolyalkoxyalkyl-, Carboxyalkyl-Rest, ein Aminoalkyl-, Alkylaminoalkyl-, Amidoalkyl-, Alkylamidoalkyl-Rest, ein homo- oder hetrocyclischer, gegebenenfalls substituierter aliphatischer oder aromatischer Kohlenwasserstoffrest mit 1 bis 60 C-Atomen, vorzugsweise 1 bis 30 C-Atomen, insbesondere 12 bis 18 C-Atomen, mit der Maßgabe, dass die Summe der C-Atome aus R¹ + R² mindestens 12 ist und
X⁻ ein salzbildendes Anion oder das Hydroxylanion ist,
verwendet wird.

2. Emulsionen gemäß Anspruch 1 ggf. in Mischung mit den nicht protonierten Alkylguanidinen, **dadurch gekennzeichnet, dass** in den Verbindungen der allgemeinen Formel (I) R¹ und R² unabhängig voneinander H oder ein gegebenenfalls Doppelbindungen enthaltender und/oder substituierter Kohlenwasserstoffrest mit 8 bis 30 C-Atomen ist.

3. Emulsionen gemäß Anspruch 1, **dadurch gekennzeichnet, dass** in den Verbindungen der allgemeinen Formel (I) R¹ und R² unabhängig voneinander H oder ein gegebenenfalls Doppelbindungen enthaltender und/oder substituierter Kohlenwasserstoffrest mit 12 bis 18 C-Atomen ist.

4. Emulsionen gemäß Anspruch 1, **dadurch gekennzeichnet, dass** in den Verbindungen der allgemeinen Formel (I) R¹ und R² ein gegebenenfalls Doppelbindungen enthaltender und/oder substituierter Kohlenwasserstoffrest mit 12 bis 18 C-Atomen ist.

5. Emulsionen gemäß Anspruch 1, **dadurch gekennzeichnet, dass** in den Verbindungen der allgemeinen Formel (I) R¹ = H und R² ein gegebenenfalls Doppelbindungen enthaltender und/oder substituierter Kohlenwasserstoffrest mit 12 bis 18 C-Atomen ist.

6. Emulsionen gemäß mindestens einem der Ansprüche 1 bis 5, **dadurch gekennzeichnet, dass** X⁻ mindestens ein Anion ist, ausgesucht aus der Gruppe der organischen oder anorganischen ein oder mehrbasischen Säuren, insbesondere der Kohlensäure, Phosphorsäure, Schwefelsäure, Essigsäure, Milchsäure, Weinsäure, Äpfelsäure, Citronensäure.

7. Verwendung der Emulsionen gemäß mindestens einem der Ansprüche 1 bis 6 zur Herstellung von kosmetischen, dermatologischen oder pharmazeutischen Zubereitungen.

8. Verwendung der Emulsionen gemäß mindestens einem der Ansprüche 1 bis 6 zur Herstellung von kosmetischen Reinigungs- und Pflegezubereitungen für Haut- und Hautanhangsgebilde, zur Herstellung von Sonnenschutzformulierungen und zur Herstellung von Desodorantien und Antitranspirantien.

9. Verwendung der Emulsionen gemäß Anspruch 1 bis 6 zur Herstellung von Zubereitungen zur Behandlung und Nachbehandlung von Geweben auf Basis natürlicher oder synthetischer Fasern wie insbesondere zur Erstaurüstung von Textilien oder, gegebenenfalls in Kombination mit weiteren quartären Verbindungen, als Wäscheweichspülmittel.

10. Verwendung der Emulsionen gemäß Anspruch 1 bis 6 zur Herstellung von Reinigungs- und Pflegemitteln für harte Oberflächen.

11. Verwendung von Alkylguanidinen gemäß mindestens einem der Ansprüche 1 bis 6 in Öl-in-Wasser- oder in Wasser-in-Öl-Emulsionen in einer Konzentration von üblicherweise 0,05 bis 10 Gew.-%, vorzugsweise 0,1 bis 5 Gew.-%, insbesondere 0,2 bis 3 Gew.-% als
- alleinige Emulgatoren in kosmetischen und pharmazeutischen Öl-in-Wasser-Emulsionen,
- Co-Emulgatoren in kosmetischen und pharmazeutischen Ö1-in-Wasser-Emulsionen,
- Co-Emulgatoren in kosmetischen und pharmazeutischen Wasser-in-Öl-Emulsionen.
